(19) Europäisches Patentamt
European Patent Office
Office européen des brevets

(11) Veröffentlichungsnummer: **0 137 372**
**B1**

(12) **EUROPÄISCHE PATENTSCHRIFT**

(45) Veröffentlichungstag der Patentschrift: **27.08.86**

(51) Int. Cl.⁴: **C 07 C 101/04**, C 07 C 99/12, C 12 P 41/00

(21) Anmeldenummer: **84111207.1**

(22) Anmeldetag: **20.09.84**

(54) **Verfahren zur Herstellung der optischen Antipoden von tert.-Leucin.**

(30) Priorität: **27.09.83 DE 3334848**

(43) Veröffentlichungstag der Anmeldung: **17.04.85 Patentblatt 85/16**

(45) Bekanntmachung des Hinweises auf die Patenterteilung: **27.08.86 Patentblatt 86/35**

(84) Benannte Vertragsstaaten: **AT BE CH DE FR GB IT LI NL SE**

(56) Entgegenhaltungen:
**AT - B - 169 327**
**DE - A - 2 105 009**
**DE - A - 2 419 838**
**DE - A - 2 732 301**
**GB - A - 1 072 876**
**GB - A - 1 369 462**

(73) Patentinhaber: **HOECHST AKTIENGESELLSCHAFT, Postfach 80 03 20, D-6230 Frankfurt am Main 80 (DE)**

(72) Erfinder: **Grabley, Susanne, Dr., Hölderlinstrasse 7, D-6240 Königstein/Taunus (DE)**
Erfinder: **Schlingmann, Merten, Dr., Schneidhainer Strasse 32a, D-6240 Königstein/Taunus (DE)**

**EP 0 137 372 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Optisch aktives tert.-Leucin, das aus dem chiralen Pool der Natur nicht zugänglich ist, besitzt als chirales Hilfsreagenz in der asymmetrischen organischen Synthese außerordentliches Interesse. Durch klassische Methoden der Racematspaltung ist es nur schwer in größeren Mengen zugänglich.

Aus der britischen Patentschrift 1 369 462 ist zwar ein Verfahren bekannt, bei dem ein Racemat der Formel

$$\begin{array}{l} R-CH-COOH \\ \quad\;\;\, | \\ \quad\;\, NHR_1 \end{array}$$

in der R eine gegebenenfalls substituierte Alkyl-, Aryl- oder Aralkylgruppe und $R_1$ eine Acylgruppe ist, für die es eine spezifische Deacylase gibt, im wäßrigen Medium mit einer Deacylase behandelt wird, die bekanntermaßen die N-Acylseitenkette eines Penicillins unter Bildung von 6-Aminopenicillansäure entfernt, wobei die freie L-Aminosäure entsteht, die von der N-Acyl-D-Aminosäure abgetrennt wird. Andererseits ist es aber aus A. Plaskie et al., The journal of Antibiotics, XXXI (1978) 783-788, insbesondere S. 786, Tabelle 2, bekannt, daß die Natur der Aminosäure einen deutlichen Einfluß auf die Geschwindigkeit dieser Reaktion hat: Wenn in der vorstehend genannten Formel $R_1$ Phenylacetyl ist, verringert sich die Geschwindigkeit der Deacylierung für R = Methyl, Ethyl und Isopropyl wie 1:0,4:0,04. Erwartungsgemäß erfolgt mit R = tert.-Butyl unter den dort genannten Bedingungen überhaupt keine Reaktion mehr.

Überraschenderweise wurde nun gefunden, daß racemisches N-Acyl-tert.-Leucin dann enzymatisch leicht gespalten wird, wenn die Acylase trägergebunden vorliegt. Die Tatsache, daß das Enzym an ein inertes Material gebunden sein kann, ist bereits in der britischen Patentschrift erwähnt. Es war jedoch nicht zu erwarten, daß eine solche Fixierung die sterische Hinderung der Reaktion derartig zurückdrängt, zumal mit der Fixierung eines Enzyms generell eine Verringerung der Aktivität verbunden ist.

Die Erfindung betrifft somit ein Verfahren zur Herstellung von L-tert.-Leucin durch enzymatische Spaltung von racemischem N-Acyl-tert.-Leucin im wäßrigen Medium mit Hilfe einer trägergebundenen Acylase. Das nicht gespaltene N-Acyl-D-tert.-Leucin kann in an sich bekannter Weise durch chemische Deacylierung, beispielsweise durch saure Hydrolyse, in das freie D-tert.-Leucin übergeführt werden oder aber nach Racemisierung wieder in den Prozeß zurückgeführt werden.

Als N-Acylrest kommen alle Acylgruppen in Betracht, die bekanntermaßen durch Acylasen abgespalten werden, wie beispielsweise Alkanoylgruppen mit 1 bis 5 Kohlenstoffatomen, Aralkanoylgruppen, vorzugsweise Phenylacetyl, und Aroylgruppen wie Benzoyl, die jeweils durch Halogen, Hydroxy, niederes Alkyl oder niederes Alkoxy substituiert sein können. Geeignete Acylreste sind literaturbekannt, beispielsweise aus der genannten britischen Patentschrift und den deutschen Offenlegungsschriften 2 352 579, 2 446 320, 2 939 269 und 3 048 612. Die entsprechenden Acylasen sind ebenfalls in diesen Druckschriften erwähnt.

Wenn zur Acylierung eine schwerflüchtige Carbonsäure verwendet wird, kann das nach Abtrennung des L-tert.-Leucins und Entfernung des Wassers zurückbleibende Gemisch aus der N-Acyl-D-Verbindung und der schwerflüchtigen Carbonsäure unmittelbar durch Erhitzen wieder racemisiert werden (EP-A-0 137371 vom gleichen Anmeldetag, entsprechend der deutschen Patentanmeldung P 33 34 849.9).

Die Fixierung des Enzyms an einen Träger ist – wie vorstehend angegeben – bereits aus der britischen Patentschrift 1 369 462 bekannt, wobei als inertes Material Cellulose, Sand oder Aluminiumoxid und als Vernetzungsmittel ein bifunktionelles Reagenz wie Glutaraldehyd erwähnt sind. Verwiesen werden kann auch auf die deutsche Offenlegungsschrift 27 32 301, die speziell die Fixierung von Penicillinacylase betrifft. Ein so hergestelltes Penicillin-G-Amidase-Präparat zeigt gegenüber N-Phenacetyl-L-tert.-Leucin eine relative Aktivität von 10 bis 15% (Penicillin-G = 100%).

Besonders vorteilhafte Träger für die Amidase sind Phenol-Formaldehyd-Harze mit freien N-H-Gruppen, wie sie als schwach basische Ionenaustauscher handelsüblich sind. An diese Träger wird das Enzym mit Hilfe eines bifunktionellen Isocyanats wie Hexamethylen-1,6-diisocyanat gebunden. Besonders vorteilhaft sind macroporöse Harze mit einem mittleren Porendurchmesser von 80 bis 100 nm. Ein unter der Handelsbezeichnung ®Duolite A7 vertriebener schwach basischer Ionenaustauscher der genannten Porengröße ergibt nach Bindung mit Hexamethylen-1,6-diisocyanat ein Penicillin-G-Amidase-Präparat mit einer relativen Aktivität von über 30% (Penicillin-G = 100%). Derart immobilisierte Acylasen, ihre Herstellung und Verwendung sind Gegenstand der EP-A-0 137371 von gleichen Anmeldetag (entsprechend der deutschen Patentanmeldung P 33 34 846.4).

Der Nachteil der gegenüber dem freien Enzym verringerten Aktivität wird bei den immobilisierten Enzymen durch eine erhöhte Standzeit mehr als aufgehoben. So konnte eine Lebensdauer von über einem Jahr und eine Halbwertszeit von mehr als 8 Monaten beobachtet werden.

Je nach Löslichkeit des Ausgangsmaterials kann die Substratkonzentration bei 0,1 bis 30, vorzugsweise 4 bis 10, Gew.-% liegen. Die Temperatur und der pH-Wert richten sich nach dem jeweiligen Enzym. Im allgemeinen liegt die Umsetzungstemperatur bei 10 bis 60° C, vorzugsweise bei 20 bis 40° C, und der pH-Wert in einem Bereich von 3 bis 9, vorzugsweise 6 bis 8.

Die Reaktion kann in einem gepufferten System oder ohne Pufferzusatz ablaufen.

Die Umsetzungsdauer ist neben der Temperatur und dem Enzymimmobilisat abhängig vom Enzym-Substrat-Verhältnis, das grundsätzlich in weiten Grenzen schwanken kann. Für kurze Reaktionszeiten von etwa 0,5 bis 5 Stunden wählt man

zweckmäßig ein Gewichtsverhältnis von Enzym zu Substrat von 1:4 bis 4:1.

Die Reaktion kann absatzweise oder kontinuierlich durchgeführt werden. Im letzteren Fall wird das immobilisierte Enzym in einem Fließbett- oder Festbett-Reaktor angeordnet. So kann das Enzymimmobilisat beispielsweise in Säulen gepackt oder in einem Schlaufenreaktor eingesetzt werden.

Verlauf und Ende der Reaktion können mit polarimetrischen oder titrimetrischen Methoden verfolgt werden. Die entstehende freie Aminosäure kann außerdem quantitativ mittels der Ninhydrin-Reaktion bestimmt werden.

Die Aufarbeitung erfolgt in an sich bekannter Weise: Das freie L-tert.-Leucin kann von der nicht angegriffenen N-Acyl-D-Verbindung und der freigesetzten Säure durch die unterschiedliche Löslichkeit, beispielsweise durch Extraktion, oder auf Grund des unterschiedlichen $pK_a$-Wertes durch Adsorption, beispielsweise an Ionenaustauschern, aufgetrennt werden. Flüchtige freigesetzte Carbonsäuren können auch durch Destillation abgetrennt werden.

Besonders bevorzugte Ausgestaltungen der Erfindung werden in den folgenden Beispielen näher erläutert. Prozentangaben beziehen sich hierbei auf das Gewicht, wenn nichts anderes angegeben ist.

*Beispiel 1*

300 g des handelsüblichen schwach basischen Ionenaustauschers Duolite A7 (Phenol-Formaldehyd-Harz mit freien N-H-Gruppen der Firma Diamond Shamrock, vgl. EP-A2-37 667, Beispiel 1) werden durch 10-minütiges Rühren in 500 ml 2N Natronlauge und anschließendes Neutralwaschen mit entionisiertem Wasser in die freie Basenform überführt. Nach 24 Stunden Trocknen bei 40° C und 50 mbar werden 100 g trockenes Harz in 200 ml wasserfreiem Dichlormethan aufgenommen, mit 10 g Hexamethylen-1,6-diisocyanat versetzt und 2 Stunden unter Luftausschluß gerührt. Man filtriert das Harz ab und wäscht es mit Dichlormethan gründlich nach.

Nach Trocknen im Exsikkator wird das aktivierte Harz in 200 ml einer wäßrigen Lösung von 10 g Penicillin-G-Acylase gegeben und 2 Stunden bei Raumtemperatur gerührt. Das Produkt wird abfiltriert und mit gesättigter Kochsalzlösung und anschließend mit entionisiertem Wasser gewaschen.

*Beispiel 2*

100 g (0,4 Mol) N-Phenacetyl-D,L-tert.-Leucin werden in 400 ml 1N Natronlauge gelöst und auf einen pH-Wert von 7,8 und ein Gesamtvolumen von 1000 ml eingestellt. Diese Reaktionslösung wird auf 37° C thermostatisiert und unter Rühren mit 100 g nach Beispiel 1 immobilisierter Penicillin-G-Amidase versetzt. Durch Titrieren mit 0,05N Natronlauge wird der pH-Wert während der Hydrolysereaktion konstant gehalten und gleichzeitig der Umsetzungsverlauf registriert. Nach 3 bis 4 Stunden wird keine Natronlauge mehr verbraucht. Nach Abfiltrieren des Enzymmaterials und Ansäuern mit konzentrierter Schwefelsäure auf pH 3 kristalisieren Phenylessigsäure und N-Phenacetyl-D-tert.-Leucin aus. Die Phenylessigsäure kann durch Extraktion mit Essigsäureethylester-Hexan aus dem Kristallisat abgetrennt werden, worauf 45 g (0,18 Mol = 90% d. Th.) N-Phenacetyl-D-tert.-Leucin vom Schmelzpunkt 163 bis 164° C, $[\alpha]_D^{20}$ 14,2° (c = 1, in Methanol) zurückbleiben.

Aus der eingeengten Mutterlauge werden durch Extraktion mit Ethanol-Wasser 23 g (0,18 Mol = 90 d. Th.) L-tert.-Leucin vom Schmelzpunkt über 250° C, $[\alpha]_D^{20}$ −9,4° (c = 1, in Wasser) isoliert.

*Beispiel 3*

Aus 50 g (0,2 Mol) N-Phenacetyl-D,L-tert.-Leucin wird entsprechend Beispiel 2 eine 10%ige Substralösung hergestellt und kontinuierlich bei 37° C durch eine Säule gepumpt, die mit 100 g Enzymimmobilisat entsprechend Beispiel 1 gefüllt ist. Im Vorratsgefäß wird der pH-Wert bei 7,8 durch Zugabe von 0,05N Natronlauge gehalten. Nach etwa 2 Stunden ist die Hydrolyse beendet. Die Aufarbeitung erfolgt nach Beispiel 2.

*Beispiel 4*

Man verfährt gemäß Beispiel 3, trennt jedoch die Freigesetzte Aminosäure wie folgt ab: Man gibt die Reaktionslösung über einen stark sauren Kationenaustauscher in der $H^+$-Form (®Dowex 50 Wx2) und desorbiert mit 5%iger wäßriger Ammoniaklösung. Aus dem Desorbat erhält man durch Vakuumtrocknung bei 80 bis 100° C 12,4 g (0,095 Mol = 95% d. Th.) L-tert.-Leucin, Schmelzpunkt über 250° C, $[\alpha]_D^{20}$ −9,44° (c = 1, in Wasser).

Aus dem Eluat des Ionenaustauschers isoliert man die Phenylessigsäure und das N-Phenacetyl-D-tert.-Leucin gemäß Beispiel 2.

*Beispiel 5*

Aus dem gemäß Beispiel 2 bis 4 erhaltenen N-Phenacetyl-D-tert.-Leucin isoliert man das D-tert.-Leucin durch saure Hydrolyse mit Salzsäure: $[\alpha]_D^{20}$ +9,0° (c = 1, in Wasser).

**Patentansprüche**

1. Verfahren zur Herstellung von L-tert.-Leucin aus N-Acyl-D,L-tert.-Leucin, dadurch gekennzeichnet, daß man das Racemat im wäßrigen Medium mit einer immobilisierten Acylase spaltet und das nicht umgesetzte N-Acyl-D-tert.-Leucin und die freigesetzte Carbonsäure abtrennt.

2. Verfahren nach Anspruch 1, dadurch gekennzeichnet, daß man aus dem N-Acyl-D-tert.-Leucin durch saure Hydrolyse das D-tert.-Leucin freisetzt.

3. Verfahren nach Anspruch 1 oder 2, dadurch gekennzeichnet, daß man als Acylverbindung die Phenacetylverbindung einsetzt.

4. Verfahren nach einem oder mehreren der vorhergehenden Ansprüche, dadurch gekennzeichnet, daß man als Acylase Penicillin-G-Amidase einsetzt.

5. Verfahren nach einem oder mehreren der vorhergehenden Ansprüche, dadurch gekennzeichnet, daß man die Acylase mit Hilfe eines Alkylendiisocyanats an ein Phenol-Formaldehyd-Harz mit freien N-H-Gruppen fixiert.

## Claims

1. A process for the preparation of L-tert.-Leucine from N-acyl-D,L-tert.-Leucine, wherein the racemate is resolved in an aqueous medium with an immobilized acylase and the unreacted N-acyl-D-tert.-Leucine and the liberated carboxylic acid are separated off.

2. The process as claimed in claim 1, wherein the D-tert.-Leucine is liberated from the N-acyl-D-tert.-Leucine by acide hydrolysis.

3. The process as claimed in claim 1 or 2, wherein the phenacetyl compound is used as the acyl compound.

4. The process as claimed in one or more of the preceding claims, wherein penicillin-G amidase is used as the acylase.

5. The process as claimed in one or more of the preceding claims, wherein the acylase is fixed with the aid of an alkylene diisocyanate to a phenol/formaldehyde resin with free N-H groups.

## Revendications

1. Procédé pour la préparation de L-tert.-leucine à partir d'acyl-D,L-tert.-leucine, caractérisé par le fait que l'on dédouble le racémique en milieu aqueux avec une acylase immobilisée et on sépare la N-acyl-D-tert.-leucine qui n'a pas réagi et l'acide carboxylique libéré.

2. Procédé selon la revendication 1, caractérisé par le fait que l'on libère la D-tert.-leucine à partir de la N-acyl-D-tert.-leucine par hydrolyse acide.

3. Procédé selon la revendication 1 ou 2, caractérisé par le fait que l'on utilise en tant que composé acylé le composé phénacétylé.

4. Procédé selon une ou plusieurs des revendications précédentes, caractérisé par le fait que l'on utilise en tant qu'acylase la pénicilline G-amidase.

5. Procédé selon une ou plusieurs des revendications précédentes, caractérisé par le fait que l'on fixe l'acylase sur une résine phénol-formaldéhyde à groupes NH libres, à l'aide d'un alkylène-diisocyanate.